# EUROPÄISCHE PATENTSCHRIFT

(11) **EP 0 393 386 B1**
(45) Veröffentlichungstag und Bekanntmachung des Hinweises auf die Patenterteilung: **11.08.1993**
(21) Anmeldenummer: 90105764.6
(22) Anmeldetag: 27.03.1990
(51) Int. Cl.: B67D 1/10, G01F 11/04

(54) **Dosiereinrichtung**
Metering device
Dispositif de dosage

(30) Priorität: 20.04.1989 DE 3913008; 13.12.1989 DE 3941103
(43) Veröffentlichungstag der Anmeldung: 24.10.1990
(73) Patentinhaber: ASM ANLAGEN UND SYSTEME FÜR MEDIZINTECHNIK GMBH, D-13581 Berlin (DE)
(72) Erfinder:
(74) Vertreter: Flosdorff, Jürgen, Dr.

(56) Entgegenhaltungen:
- DE-A- 2 330 382
- FR-A- 2 441 836

## Beschreibung

Die Erfindung betrifft eine Dosiereinrichtung zum Zuführen zweier Flüssigkeiten, von denen wenigstens eine dosiert sein kann. Grundsätzlich ist die Dosiereinrichtung nicht nur für zwei Flüssigkeiten, sondern auch für zwei gasförmige Medien verwendbar, wobei im folgenden jedoch stets von Flüssigkeiten gesprochen wird.

Eine Dosiereinrichtung der betrachteten Art kann beispielsweise im Bereich der Medizintechnik zum Desinfizieren oder Reinigen von Geräten eingesetzt werden. Eine spezielle Verwendungsmöglichkeit ist im Zusammenhang mit Dialysemaschine gegeben, die nach einer Bikarbonat-Dialyse mit Zitronensäure entkalkt werden. Hierzu wird üblicherweise ein separates Zitronensäure-Reservoir an die Maschine angeschlossen. Nach Abschluß des Entkalkungsvorgangs wird dieses Zitronensäure-Reservoir abgehängt, woraufhin nach dem Umstecken der Spülkonnektoren der Maschine ein Klarspülprogramm gestartet wird. Diese beiden voneinander unabhängigen Programme erstrecken sich über eine verhältnismäßig große Zeitspanne von 30 bis 60 Minuten, wobei eine Bedienungsperson erforderlich ist, um die zugehörigen Handgriffe an der Maschine auszuführen. Durch die beträchtliche Zeitdauer der Entkalkung der Dialysemaschine und die dabei erforderlichen Maßnahmen seitens der Bedienungsperson ist ein solcher Vorgang mit beträchtlichen Kosten verbunden.

Der vorliegenden Erfindung liegt die Aufgabe zugrunde, ein Gerät anzugeben, mit dem zwei Flüssigkeiten, von denen wenigstens eine dosiert ist, auf einfache Weise beispielsweise in einen Flüssigkeitskreislauf einer Maschine eingeführt werden können.

Diese Aufgabe wird erfindungsgemäß durch die im Kennzeichen des Patentanspruchs 1 angegebenen Merkmale gelöst. Vorteilhafte Weiterbildungen der Erfindung sind in den Unteransprüchen gekennzeichnet.

Die erfindungsgemäße Dosiereinrichtung enthält einen Hohlzylinder, an dessen Innenwand ein verschieblicher Kolben flüssigkeitsdicht anliegt, der eine vordere und eine rückwärtige Zylinderkammer begrenzt, deren Volumen durch die Bewegung des Kolbens veränderbar ist. Im rückwärtigen Endbereich hat der Hohlzylinder eine Flüssigkeits-Eintrittsöffnung, durch die beispielsweise Wasser eintreten kann, und im vorderen Endbereich eine Öffnung, durch die eine weitere Flüssigkeit in die vordere Zylinderkammer einfüllbar ist, wobei dies beispielsweise ein Desinfektionsmittel oder ein Entkalkungsmittel sein kann. Die vordere Öffnung dient ferner dem Austritt beider Flüssigkeiten, wobei zudem eine Flüssigkeit-Durchlaßeinrichtung von der hinteren zur vorderen Zylinderkammer vorgesehen ist, die in einer vorgegebenen, vorzugsweise vorgeschobenen Position des Kolbens infolge eines auf die rückwärtige Seite des Kolbens einwirkenden Drucks der zugeordneten Flüssigkeit oder eines auf die vordere Seite des Kolben einwirkenden Sogs den Austritt der in der rückwärtigen Zylinderkammer befindlichen Flüssigkeit in die vordere Zylinderkammer und aus der vorderen Öffnung des Hohlzylinders heraus zuläßt.

Vorzugsweise wird die zweite Flüssigkeit mittels einer automatischen Füllvorrichtung unter Druck in einer vorbestimmten Menge durch die vordere Öffnung in die vordere Zylinderkammer eingefüllt, wobei die Öffnung durch ein Ventil schließbar sein kann, das unter Druck in beiden Richtungen öffnen kann.

Damit die vordere Zylinderkammer der Dosiereinrichtung ohne zusätzliche Handgriffe stets wieder mit einer Flüssigkeit befüllt werden kann, wird vorzugsweise im Zusammenhang mit der Verwendung einer automatischen Füllvorrichtung vorgeschlagen, daß zwischen dem vorderen Hohlzylinderboden und dem Kolben eine Federeinrichtung angeordnet ist, vorzugsweise in Form einer in axialer Richtung verlaufenden Schraubenfeder, die komprimiert wird, wenn der Kolben in dem Bereich der Flüssigkeitsdurchlaßeinrichtung vorgeschoben wird, so daß die Federkraft den Kolben wieder in einen rückwärtigen Bereich zurückdrückt, in dem dieser dicht an der Zylinderinnenwand anliegt, wenn der Kolben nicht mehr durch Flüssigkeitsdruck in Vorschubrichtung beaufschlagt ist. Damit wird nach Beendigung eines Flüssigkeitszuführvorganges der Kolben automatisch in eine Position zurückgedrückt, in der die beiden Zylinderkammern voneinander getrennt sind, so daß die vordere Kammer wieder mit einer Flüssigkeit befüllt werden kann.

Die Durchlaßeinrichtung zwischen der rückwärtigen und der vorderen Zylinderkammer kann durch mehrere axiale Bohrungen gebildet sein, die von außen durch den vorderen Zylinderboden hindurch Umfangsabschnitte der Innenwand des inneren vorderen Endabschnitts des Hohlzylinders wegschneiden. Anschließen kann der Zylinderboden dadurch geschlossen werden, daß zylindrische Füllstücke eingesetzt, vorzugsweise eingeklebt werden, die den vorderen Zylinderboden wieder schließen. Damit verbleiben lediglich im vorderen Endabschnitt der Zylinderinnenwand Aufweitungen, an denen die in der hinteren Zylinderkammer befindliche, unter Druck stehende Flüssigkeit den vorzugsweise durch einen O-Ring seitlich abgedichteten Kolben umströmen kann. Diese Herstellungsmethode ist besonders einfach und kostengünstig durchführbar.

An der Dosiereinrichtung können seitlich zwei Konnektoren angebracht sein, mit denen die Dosiereinrichtung beispielsweise an einem Dialysegerät befestigt werden kann, wobei durch einen der Konnektoren Wasser in die hintere Zylinderkammer eingeführt werden kann.

Mit der erfindungsgemäßen Dosiereinrichtung kann aber auch von Hand nach Art einer herkömmlichen Spritze durch die vordere Öffnung eine genau definierte Menge einer Flüssigkeit in die vordere Zylinderkammer eingesaugt werden, wenn der Kolben mit einer aus dem Hohlzylinder herausragenden Stange versehen ist, die in eine rückwärtige Position gebracht werden kann, die das angesaugte Volumen der Flüssigkeit festlegt. Anschließend kann die erste, unter Druck stehende Flüssigkeit (z.B. Wasser) durch die rückwärtige Eintrittsöffnung in die hintere Zylinderkammer eingelassen werden, wobei durch denDruck dieser zweiten Flüssigkeit der Kolben vorgeschoben wird, der hierbei die Flüssigkeit aus der vorderen Zylinderkammer ausbringt. In einer vorbestimmten Position des Kolbens wird der Flüssigkeits-Durchlaß freigegeben, der die bis dahin währende Funktion des Kolbens als Trennwand zwischen vorderer und hinterer Kammer aufhebt und den Austritt der in der rückwärtigen Zylinderkammer befindlichen Flüssigkeit in die vordere Zylinderkammer zuläßt. Anschließend tritt die erste Flüssigkeit aus der vorderen Öffnung des Hohlzylinders aus, wobei sie zudem den Rest der noch in der vorderen Zylinderkammer befindlichen anderen Flüssigkeit ausspült.

Auf diese Weise können die beiden Flüssigkeiten in einem bestimmten Mengenverhältnis beispielsweise einer Maschine zugeführt werden, in der die beiden Flüssigkeiten miteinander vermischt werden können. Dabei kann auch die zunächst durch die rückwärtige Zylinderkammer zugeführte erste Flüssigkeit auf geeignete Weise dosiert sein.

Wenn der Durchlaß von der hinteren in die vordere Zylinderkammer erst dann freigegeben ist, wenn sich der Kolben in der vorgeschobenen Position befindet, ist die zunächst in der vorderen Zylinderkammer befindliche Flüssigkeit bereits nahezu vollständig aus der vorderen Kammer ausgestoßen (und der nachgeordneten Aufnahmeeinrichtung zugeführt), bevor die Flüssigkeit aus der rückwärtigen Zylinderkammer austreten kann. Hierbei kann die Ausgestaltung so getroffen sein, daß der Kolben an der vorderen Stirnwand des Hohlzylinders anliegt, wenn der Durchlaß geöffnet wird, so daß die erste Flüssigkeit direkt durch die vordere Öffnung den Hohlzylinder verläßt.

Es kann aber auch zweckmäßig sein, die Anordnung so zu treffen, daß der Durchlaß von der hinteren in die vordere Zylinderkammer bereits in einer rückwärtigen Position des Kolbens freigegeben wird, so daß sich die beiden Flüssigkeiten bereits in der vorderen Zylinderkammer in einem gewissen Umfang miteinander vermischen können.

An der vorderen Stirnfläche des Hohlzylinders kann im Bereich der Öffnung ein Anschlußstutzen angebracht sein, dessen Durchgangsbohrung mit der Zylinderöffnung in Verbindung steht. Dieser Anschlußstutzen kann dazu dienen, die erfindungsgemäße Dosiereinrichtung beispielsweise mit dem Konzentrateingang einer Dialysemaschine zu verbinden, um nach Zufuhr von Zitronensäure aus der vorderen Zylinderkammer und von zunächst in die hintere Zylinderkammer eingeleitetem Wasser einen Klarspüldurchlauf mit gleichzeitiger Entkalkung einzuleiten.

Nach einem weiteren Vorschlag der Erfindung kann die die rückwärtige Stirnwand des Hohlzylinders durchgreifende Zugstange ein Hohlrohr sein, das beispielsweise über eine Schlauchtülle oder eine andere geeignete Befestigung mit einer Leitung verbindbar ist, die mit einer Quelle der ersten Flüssigkeit in Verbindung steht.

Bei dieser Ausgestaltung der Erfindung tritt die erste Flüssigkeit demnach nur in das mit dem Kolben verbundene Hohlrohr, nicht jedoch in die hintere Zylinderkammer ein, was den Vorteil hat, daß die Zugstange nicht flüssigkeitsdicht gegenüber der Durchgangsbohrung, die sie durchgreift, abgedichtet sein muß. Da der auf denKolben wirkende Druck der ersten Flüssigkeit auf eine Fläche beschränkt ist, die durch die Innenabmessungen des Hohlrohres vorgegeben ist, muß der Druck der ersten Flüssigkeit verhältnismäßig groß sein, um den Kolben vorzuschieben. Alternativ hierzu kann der Vorschub jedoch auch durch einen auf die Vorderseite des Kolbens einwirkenden Sog bewirkt werden, der z.B. durch eine Pumpe erzeugt werden kann.

Der Durchlaß kann dadurch gebildet sein, daß der Kolben eine mit einem Absperrglied versehene Durchgangsbohrung aufweist, die den Innenraum des Hohlrohrs mit der vorderen Zylinderkammer verbindet und den Austritt der Flüssigkeit zuläßt, wenn das Absperrglied in eine Öffnungsstellung versetzt ist. Alternativ hierzu kann die Durchgangsbohrung des Kolbens die hintere Zylinderkammer mit der vorderen Zylinderkammer verbinden.

Das Absperrglied kann ein federbelasteter Verschlußkörper beispielsweise in Form einer Kugel sein, die von einer Feder in Richtung der vorderen Stirnwand des Hohlzylinders in die Verschlußstellung, d.h. gegen einen Ventilsitz, gedrückt wird. Dieses Ventil kann in der vorgeschobenen Position des Kolbens dadurch geöffnet werden, daß der Verschlußkörper auf einen feststehenden Vorsprung aufläuft, der den Verschlußkörper gegendie Kraft der Feder in die Öffnungsstellung zurückdrückt. Der Vorsprung kann ein im Bereich der vorderen Öffnung des Hohlzylinders angebrachter Aufdrückdorn sein, der mit wenigstens einer Durchgangsbohrung versehen sein kann, die mit der Austrittsöffnung aus dem Hohlzylinderin Verbindung steht.

In einer alternativen Ausgestaltung der Erfindung ist vorzugsweise in der rückwärtigen Stirnwand des Hohlzylinders, gegebenenfalls neben einer von einer vorerwähnten Zugstange durchgriffenen Durchgangsbohrung, eine weitere Öffnung zum Eintritt der ersten Flüssigkeit in die rückwärtige Zylinderkammer vorgesehen. Diese Öffnung kann mit einem Anschlußstutzen für eine Flüssigkeitsleitung, beispielsweise einem Schlauch, versehen sein. Da in diesem Fall die erste Flüssigkeit in die rückwärtige Zylinderkammer tritt, muß bei Anordnung einer Zugstange die von dieser durchgriffene Durchgangsbohrung mit einer Dichtung versehen sein, die den Austritt der Flüssigkeit an dieser Stelle verhindert.

Um den Austritt der ersten Flüssigkeit aus der rückwärtigen Zylinderkammer in die vordere Zylinderkammer zu ermöglichen, kann, wie bereits oben erwähnt, im Bereich des vorderen Endabschnitts des Hohlzylinders wenigstens ein Umfangsabschnitt der Innenwand des Hohlzylinders aufgeweitet sein. Die Aufweitung der Innenwand kann durch eine oder mehrere Aussparungen bewerkstelligt sein, oder durch eine Abstufung des gesamten Umfangs der Innenwand mit entsprechender Vergrößerung des Innendurchmessers. Im letzteren Falle ist der Kolben zweckmäßigerweise mit abgeschrägten Außenwänden versehen, damit er sich an der Abstufung nicht festsetzen kann.

Wie bereits erwähnt, können mit der erfindungsgemäßen Dosiereinrichtung zwei FLüssigkeiten auf einfache Weise dosiert und beispielsweise einer nachgeordneten Dialysemaschine zugeführt werden. Zum Entkalken der Dialysemaschine wird zunächst die vordere Zylinderkammer beispielsweise nach Art einer herkömmlichen Spritze oder mittels einer automatischen Füllvorrichtung mit Zitronensäure gefüllt, und zwar in einer exakt vorgegebenen Menge. Der Ausgang der erfindungsgemäßen Dosiereinrichtung, vorzugsweise der hierzu vorgesehene Maschinen-spezifische Anschlußstutzen, wird mit dem Konzentrateingang der Dialysemaschine über geeignete Konnektoren verbunden. Der rückwärtige Flüssigkeitseingang der Dosiereinrichtung wird ebenfalls mittels geeigneter Konnektoren mit dem Spüleingang verbunden. Danach wird das Spülprogramm oder ein Heißreinigungsprogramm gestartet.

In der ersten Phase dieses Spülprogramms wird die Dosiereinrichtung entleert, d.h. die Zitronensäure aus der vorderenZylinderkammer ausgebracht, wobei im entleerten Zustand, d.h. in der vorgeschobenen Position des Kolbens, der Spüleingang mit dem Konzentrateingang der Dialysemaschine verbunden ist, so daß alle im Flüssigkeitskreislauf befindlichen Teile durchgespült werden. Hierdurch ergibt sich ein erheblich verringerter Bearbeitungsaufwand für eine Bedienungsperson.

Nachfolgend werden drei Ausführungsformen der Erfindung anhand der Zeichnung beschrieben. Dabei zeigen:
- Fig. 1: eine erste Ausführungsform der erfindungsgemäßen Dosiereinrichtung in einer rein schematischen Schnittansicht;
- Fig. 2: eine zweite Ausführungsform der Dosiereinrichtung in einer entsprechenden Darstellung;
- Fig. 3: einenLängsschnitt durch eine dritte Dosiereinrichtung gemäß der Erfindung;
- Fig. 4: den vorderen Zylinderboden der Dosiereinrichtung gemäß Figur 3 nach einem ersten Arbeitsschritt zur Herstellung der Flüssigkeit-Durchlaßeinrichtung und
- Fig. 5: den Bereich des Kolbens der Dosiereinrichtung gemäß Fig. 3 in einer vergrößerten Darstellung.

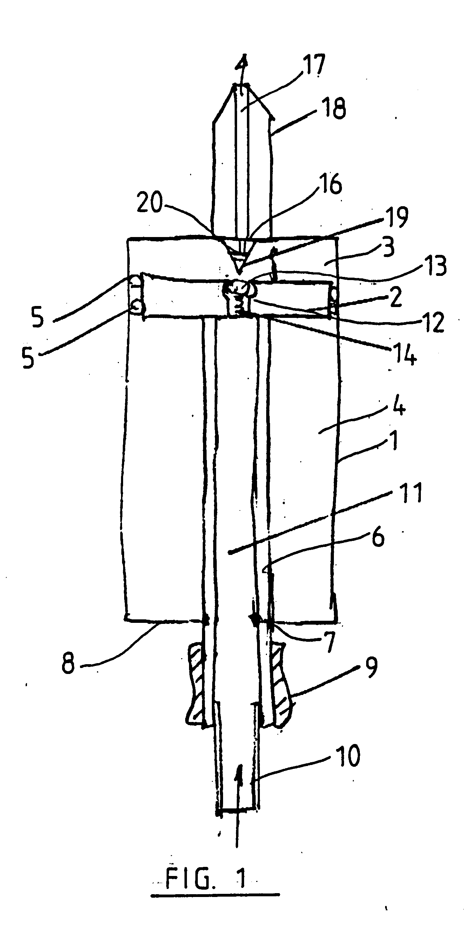

Die in Figur 1 dargestellte Dosiereinrichtung enthält einen Hohlzylinder 1, in dem ein Kolben 2 verschieblich geführt ist, der hierbei den Innenraum des Hohlzylinders 1 in eine vordere Zylinderkammer 3 und eine rückwärtige Zylinderkammer 4 unterteilt. Der Kolben 2 liegt mittels Dichtungen 5 flüssigkeitsdicht an der Innenwand des Hohlzylinders 1 an.

An der Rückseite des Kolbens 2 ist als Zugstange ein hohles Rohr 6 angesetzt, das eine Durchgangsbohrung 7 in der rückwärtigen Stirnwand 8 des Hohlzylinders 1 durchgreift. Der rückwärtige Endabschnitt des hohlen Rohres 6 ist mit einem Griffstück 9 versehen.

Das Rohr 6 ist mit einer Flüssigkeitszufuhrleitung 10 verbunden, die ihrerseits mit einer nicht dargestellten Quelle für eine unter Druck stehende Flüssigkeit wie z.B. Wasser in Verbindung steht, so daß diese Flüssigkeit in den Innenraum 11 des Rohres 6 eingeführt werden kann.

Der Kolben 2 ist mit einem Ventil 12 versehen, das im geöffneten Zustand den Durchfluß der in dem Innenraum 11 des Rohres 6 befindlichen Flüssigkeit in die vordere Zylinderkammer 3 freigibt. Das Ventil 12 ist mit einem kugelförmigen Ventilkörper 13 versehen, der von einer Feder 14 gegen einen Ventilsitz gedrückt wird.

In der vorderen Stirnwand 15 des Hohlzylinders 1 ist eine Öffnung 16 ausgebildet, die mit einer Durchgangsbohrung 17 eines an der Stirnwand 15 angesetzten Anschlußstutzens 18 in Verbindung steht. Durch diese Durchgangsbohrung 17 kann die in der vorderen Zylinderkammer 3 befindliche Flüssigkeit die Dosiereinrichtung verlassen.

An der Innenseite der Stirnwand 15 ist ein Dorn 19 angesetzt, der mit Kanälen 20 versehen ist, die den Innenraum der vorderen Zylinderkammer 3 mit der Öffnung 16 verbinden. Der Dorn 19 ist auf den Ventilkörper 13 ausgerichtet, der beim Vorschub des Kolbens 2 auf den Dorn 19 auftrifft, der hierbei das Ventil gegen die Kraft der Feder 14 öffnet.

Zur exakt dosierten Flüssigkeitszufuhr wird zunächst eine Flüssigkeit durch die Durchgangsbohrung 17 des Anschlußstutzens 18 in die vordere Zylinderkammer 3 eingesaugt, indem der Kolben 2 mittels des Rohres 6 nach Art einer herkömmlichen Spritze über eine vorgegebene Wegstrecke zurückgezogen wird, die das angesaugte Volumen der Flüssigkeit festlegt. Daraufhin wird der Anschlußstutzen 18 der Dosiereinrichtung beispielsweise mittels geeigneter Konnektoren mit dem Konzentrateingang einer Dialysemaschine verbunden.

Durch die Schlauchleitung 10 wird nun dem Innenraum 11 des Rohres 6 eine weitere Flüssigkeit zugeführt, die unter Druck steht und deren Volumen durch geeignete Mittel ebenfalls vorbestimmt sein kann. Infolge des Drucks der zweiten Flüssigkeit wird eine Vorschubkraft auf den Kolben 2 ausgeübt, wodurch dieser (bei entsprechend großem Flüssigkeitsdruck) vorgeschoben wird und die in der vorderen Zylinderkammer 3 befindliche Flüssigkeit aus der Dosiereinrichtung ausstößt.

Wenn der Kolben soweit vorgeschoben ist, daß der Ventilkörper 13 auf den Dorn 19 aufläuft, wird das Ventil 12 geöffnet, so daß die zweite Flüssigkeit über die vordere Zylinderkammer 3, die Bohrungen 20 des Dorns 19 unddie Durchgangsbohrung 17 des Anschlußstutzens 18 aus der Dosiereinrichtung austritt. Hierbei wird die noch in der vorderen Zylinderkammer 3 befindliche Restflüssigkeit mit ausgespült.

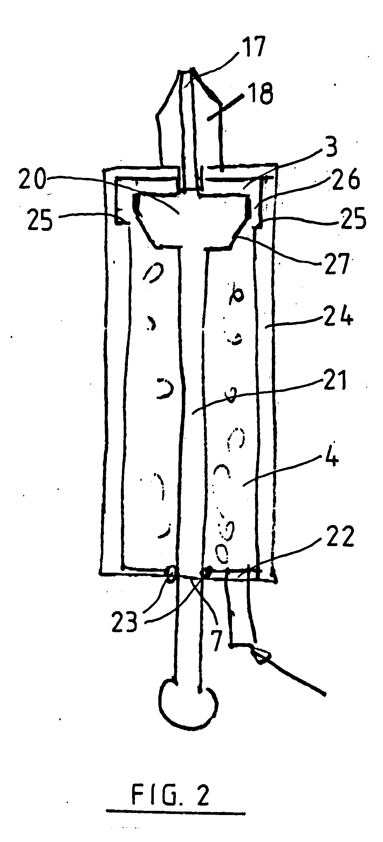

Bei der in Figur 2 dargestellten Dosiereinrichtung wird die erste Flüssigkeit nicht dem Innenraum der mit dem Kolben 20 verbunden Zugstange 21, sondern durch eine weitere Öffnung 22 der hinteren Zylinderkammer 4 zugeführt. Bei dieser Ausführungsform ist die hintere Zylinderkammer 4 mittels Dichtungen 23 an der Durchgangsbohrung 7 flüssigkeitsdicht gegenüber der Zugstange 21 abgedichtet.

Die Innenwand des Hohlzylinders 24 ist im vorderen Endbereich durch eine Abstufung 25 derart aufgeweitet, daß in der vorgeschobenen Position des Kolbens 20 ein Durchlaß 26 von der hinteren Zylinderkammer 4 zur vorderen Zylinderkammer freigegeben ist, so daß die rückwärtige Flüssigkeit durch die Durchgangsbohrung 17 des Anschlußstutzens 18 die Dosiereinrichtung verlassen kann.

Der Kolben 20, der in den übrigen Bereichen flüssigkeitsdicht an der Innenwand des Zylinders 24 anliegt, enthält an seiner Rückseite geneigte Flächen 27, so daß der Kolben 20 trotz der Abstufung 25 stets glatt zurückgezogen werden kann.

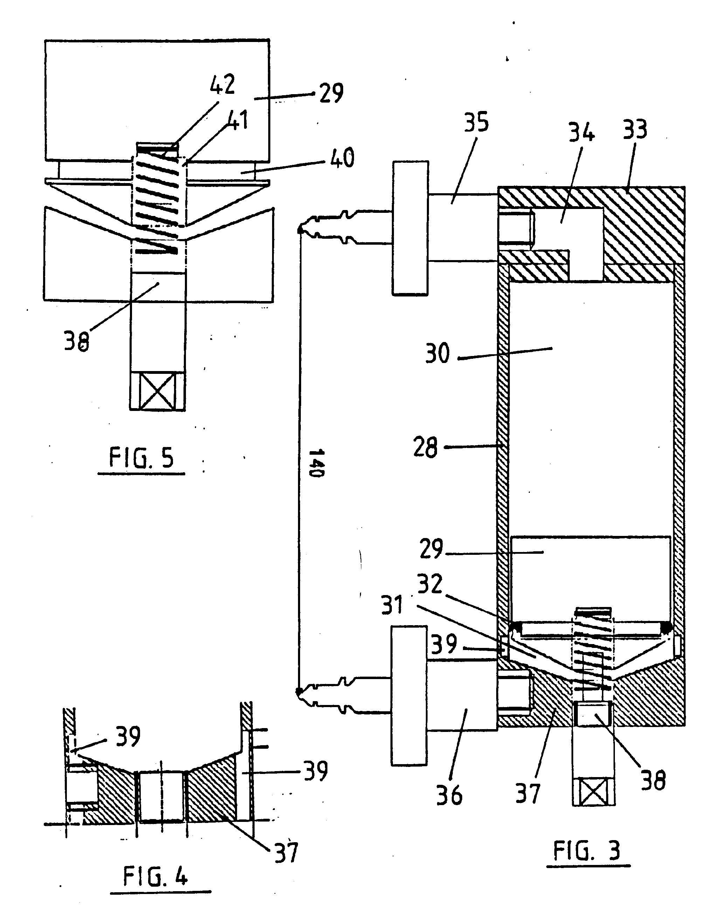

Die in Figur 3 abgebildete, bevorzugte Dosiereinrichtung enthält einen Hohlzylinder 28, in dem ein Kolben 29 hin- und herbewegbar ist, der den Innenraum des Hohlzylinders 28 in einen hinteren und einen vorderen Zylinderraum 30, 31 unterteilt. Der Kolben 29 ist mittels eines O-Ringes 32 aus Silikon gegenüber der Innenwand des Zylinders 28 abgedichtet.

Der in der Figur obere Zylinderboden 33 ist mit der rohrförmigen Wand des Zylinders 28 verklebt und enthält die obere bzw. rückwärtige Eintrittsöffnung 34 für eine erste Flüssigkeit, z.B. Wasser, die durch einen eingesteckten Konnektor 35 zugeführt wird, mit dem die Dosiereinrichtung beispielsweise an einem Dialysegerät befestigbar ist. Hierzu ist ferner am anderen axialen Ende ein weiterer seitlicher Konnektor 36 vorgesehen, der nur der Befestigung, nicht jedoch einer Flüssigkeitszufuhr dient. Außerdem können die beiden Konnektoren 35, 36 einen elektrischen Kontakt herstellen, mit dem dem Dialysegerät signalisiert werden kann, daß beispielsweise ein chemischer Desinfektionsvorgang ausgeführt werden soll.

In dem unteren (vorderen) Zylinderboden 37 befindet sich eine zentrale vordere Öffnung 38, durch die eine zweite Flüssigkeit in die vordere Zylinderkammer 31 eingeführt werden kann. Die Öffnung 38 ist mittels eines Ventils verschlossen, das unter Druck in beiden Richtungen öffnen kann. Die vordere Öffnung 38 dient ferner dem Austritt der in der vorderen Zylinderkammer 31 befindlichen zweiten Flüssigkeit sowie dem Austritt der ersten Flüssigkeit der hinteren Zylinderkammer 30, wenn diese den Kolben 29 umströmen kann.

Hierzu ist in dem vorderen (unteren) Endbereich der Innenwand des Hohlzylinders 28 eine Flüssigkeits-Durchlaßeinrichtung in Form von mehreren kreisförmigen Bohrungen 39 vorgesehen. Wenn der Kolben 29 so weit vorgeschoben ist, daß sich sein Dichtring 32 im Bereich der durch die Bohrungen 39 geschaffenen Aufweitung befindet, kann die in der hinteren Zylinderkammer 30 befindliche Flüssigkeit den Dichtring 32 radial außen umströmen und gelangt in die vordere Zylinderkammer 31, aus der sie die restliche Flüssigkeitsmenge (z.B. Desinfektionsmittel) ausführt und selbst aus der Öffnung 38 austritt.

Aus Fig. 4 ist zu ersehen, daß die Flüssigkeits-Durchlaßeinrichtung dadurch ausgebildet ist, daß von der unteren Stirnseite her mehrere zylindrische Bohrungen ausgeführt werden, die anschließend im Bereich des Zylinderbodens 37 wieder durch entsprechend geformte Füllstücke geschlossen werden, die beispielsweise eingeklebt werden. Danach verbleiben noch die in Fig. 3 abgebildeten Aussparungen in der Zylinderinnenwand.

Der in Figur 5 vergrößert dargestellte Kolben 29 enthält eine Ringnut 40 zur Aufnahme des O-Ringes 32 und eine Bohrung 41 zur Aufnahme der Schraubenfeder 42, deren Gegensitz durch das Ventil der Öffnung 38 gebildet ist. Wenn der Kolben eine derart vorgeschobene Position erreicht hat, daß sich sein O-Ring 32 im Bereich der Aufweitungen 39 befindet, ist die Schraubenfeder 42 so weit zusammengedrückt, daß sie den Kolben 29 selbsttätig wieder aus dem Bereich der Aufweitungen 39 zurückdrückt, wenn dieser nicht mehr von einem Flüssigkeitsdruck beaufschlagt ist. Damit ist wieder eine flüssigkeitsdichte Trennung zwischen der hinteren und der vorderen Zylinderkammer 30, 31 geschaffen, so daß die vordere Kammer 31 wieder befüllt werden kann, ohne daß die Flüssigkeit zur hinteren Kammer 30 strömt.

Wie die Figuren 3 und 5 zeigen, hat der Kolben an seiner (unteren) Vorderseite die Form eines Kegelstumpfes, während der Zylinderboden 37 an seiner Innenseite eine negative Kegelstumpfform hat. Dabei ist der Neigungswinkel des Kolbens größer als derjenige des Zylinderbodens. Durch diese Formgebung ist das glatte Ausführen der in der vorderen Zylinderkammer 31 befindlichen Flüssigkeit sowie das Ausströmen der Flüssigkeit aus der hinterenZylinderkammer erleichtert.

Das erfindungsgemäße Dosiergerät ist allgemein zum Dosieren von flüssigen oder gasförmigen Medien geeignet, wobei der Kolben nicht nur durch Überdruck in der rückwärtigen Zylinderkammer, sondern auch durch Unterdruck in der vorderen Zylinderkammer vorgeschoben werden kann.

## Patentansprüche

1. Dosiereinrichtung zum Zuführen zweier Flüssigkeiten, von denen wenigstens eine dosiert ist,
**gekennzeichnet durch**
einen Hohlzylinder (1, 24, 28), an dessen Innenwand ein verschieblicher Kolben (2, 20, 29) flüssigkeitsdicht anliegt, der eine vordere und eine rückwärtige Zylinderkammer (3, 4; 31, 30) begrenzt, eine Eintrittsöffnung (7, 22, 34) für die erste Flüssigkeit im rückwärtigen Bereich des Hohlzylinders und eine Öffnung (16, 38) im vorderen Bereich des Hohlzylinders, durch die die zweite, dosierte Flüssigkeit in die vordere Zylinderkammer (3, 31) einfüllbar ist und aus der die beiden Flüssigkeiten infolge des Vorschubs des Kolbens, der durch auf die rückwärtige Seite des Kolbens einwirkenden Druck der ersten Flüssigkeit oder auf die vordere Seite des Kolbens einwirkenden Sog hervorgerufen wird, austreten können, und eine Durchlaßeinrichtung (12, 25, 39) für die erste Flüssigkeit, die in einer vorgegebenen Position des Kolbens den Austritt der ersten Flüssigkeit in die vordere Zylinderkammer (3, 31) bzw. aus der vorderen Öffnung (16, 38) des Hohlzylinders zuläßt.

2. Dosiereinrichtung nach Anspruch 1,
dadurch gekennzeichnet, daß der Durchlaß in die vordere Zylinderkammer in der vorgeschobenen Position des Kolbens (2, 20, 29) freigegeben ist.

3. Dosiereinrichtung nach Anspruch 1 oder 2,
dadurch gekennzeichnet, daß zwischen dem vorderen Hohlzylinderboden (37) und dem Kolben (29) eine Federeinrichtung (42) angeordnet ist, die komprimiert wird, wenn der Kolben in den Bereich der Durchlaßeinrichtung (39) vorgeschoben wird, und deren Federkraft den Kolben wieder in einen Bereich zurückdrückt, in dem dieser dicht an der Zylinderinnenwand anliegt, wenn der Kolben nicht mehr in Vorschubrichtung durch Flüssigkeitsdruck oder -sog beaufschlagt ist.

4. Dosiereinrichtung nach einem der Ansprüche 1 bis 3,
dadurch gekennzeichnet, daß mit dem Kolben (2, 20) eine Zugstange (6, 21) verbunden ist, die in der vorgeschobenen Position des Kolbens mit ihrem rückwärtigen Endabschnitt aus einer Durchgangsbohrung (7) in der rückwärtigen Stirnwand (8) des Hohlzylinders (1, 24) herausragt.

5. Dosiereinrichtung nach Anspruch 4,
dadurch gekennzeichnet, daß die die rückwärtige Stirnwand (8) das Hohlzylinders (1) durchgreifende Zugstange ein hohles Rohr (6) ist, das mit einer Leitung (10) verbindbar ist, die mit einer Quelle der ersten Flüssigkeit in Verbindung steht.

6. Dosiereinrichtung nach einem der Ansprüche 1 bis 5,
dadurch gekennzeichnet, daß der Kolben (2) eine mit einem Absperrglied (13) versehene Durchgangsbohrung aufweist, die den Durchtritt der ersten Flüssigkeit in die vordere Zylinderkammer (3) zuläßt, wenn das Absperrglied (13) in eine Öffnungsstellung versetzt ist, daß das Absperrglied ein Verschlußkörper (13) ist, der von einer Federeinrichtung (14) in Richtung der vorderen Stirnwand (15) des Hohlzylinders (1) in die Verschlußstellung beaufschlagt ist, und daß in der vorgeschobenen Position des Kolbens (2) der Verschlußkörper (13) auf einen feststehenden Vorsprung (19) aufläuft, der den Verschlußkörper in die Öffnungsstellung drückt.

7. Dosiereinrichtung nach Anspruch 6,
dadurch gekennzeichnet, daß der Vorsprung ein im Bereich der vorderen Öffnung (16) des Hohlzylinders (1) angebrachter Dorn (19) ist, der wenigstens eine Durchgangsbohrung (20) aufweist, die mit der Öffnung (16) in Verbindung steht.

8. Dosiereinrichtung nach einem der Ansprüche 1 bis 7,
dadurch gekennzeichnet, daß eine Öffnung (22, 34) zum Eintritt der ersten Flüssigkeit in die rückwärtige Zylinderkammer (4, 30) ausgebildet ist.

9. Dosiereinrichtung nach Anspruch 4 und 8,
dadurch gekennzeichnet, daß die von der Zugstange (21) durchgriffene Durchgangsbohrung (7) in der rückwärtigen Stirnwand des Hohlzylinders (24) flüssigkeitsdicht gegenüber der Zugstange abgedichtet ist.

10. Dosiereinrichtung nach einem der Ansprüche 1 bis 4, 8 und 9,
dadurch gekennzeichnet, daß im Bereich des vorderen Endabschnitts des Hohlzylinders (24, 28) wenigstens ein Umfangsabschnitt der Innenwand des Hohlzylinders aufgeweitet ist, so daß in diesem Bereich ein Durchlaß (26, 39) am Rand des Kolbens (20, 29) freigegeben ist.

## Claims

1. A metering device for supplying two fluids, of which at least one is supplied in metered amounts,
**characterized** **by**
a hollow cylinder (1, 24, 28) on the inner wall of which a displaceable piston (2, 20, 29) rests in fluid-tight fashion, said piston defining front and rear cylinder chambers (3, 4; 31, 30), an entrance port (7, 22, 34) for the first fluid in the rear portion of said hollow cylinder and a port (16, 38) provided in the front portion of said hollow cylinder, through which said second, metered fluid can be filled into said front cylinder chamber (3, 31) and from which the two fluids can exit due to the advance of said piston which is caused by pressure of said first fluid which acts on the rear side of said piston or by suction acting on the front side of said piston, and passage means (12, 25, 39) for said first fluid which in a predetermined position of said piston permits said first fluid to exit into said front cylinder chamber (3, 31) or from said front port (16, 38) of said hollow cylinder.

2. A metering device according to claim 1,
characterized in that passage into said front cylinder chamber is permitted in the advanced position of said piston (2, 20, 29).

3. A metering device according to claim 1 or 2,
characterized in that the front bottom (37) of said hollow cylinder and said piston (29) have arranged thereinbetween a spring means (42) which is compressed when said piston is advanced into the area of passage means (39) and whose resilient force presses said piston back into an area in which said piston tightly rests on the inner wall of said cylinder when said piston is no longer acted upon by fluid pressure or suction in the advance direction.

4. A metering device according to any one of claims 1 to 3,
characterized in that said piston (2, 20) has connected thereto a connecting rod (6, 21) which in the advanced position of said piston projects with its rear end section from a through hole (7) in the rear end wall (8) of said hollow cylinder (1, 24).

5. A metering device according to claim 4,
characterized in that said connecting rod extending through the rear end wall (8) of said hollow cylinder (1) is a hollow tube (6) which is connectable to a conduit (10) communicating with a source of first fluid.

6. A metering device according to any one of claims 1 to 5,
characterized in that said piston (2) has a through hole provided with a shut-off member (13), which permits passage of said first fluid into said front cylinder chamber (3) when said shut-off member (13) is moved into an opening position, that said shut-off member is a closing body (13) which is acted upon by a spring means (14) towards the front end wall (15) of said hollow cylinder (1) into the closing position, and that in the advanced position of said piston (2) said closing body (13) abuts on a stationary projection (19) which presses said closing body into said opening position.

7. A metering device according to claim 6,
characterized in that said projection is a mandrel (19) which is provided in the area of said front port (16) of said hollow cylinder (1) and which includes at least one through hole (20) communicating with said port (16).

8. A metering device according to any one of claims 1 to 7,
characterized in that a port (22, 34) is formed for the entry of said first fluid into said rear cylinder chamber (4, 30).

9. A metering device according to claims 4 and 8,
characterized in that said through hole (7) through which said connecting rod (21) extends is sealed in fluid-tight fashion relative to said connecting rod In the rear end wall of said hollow cylinder (24).

10. A metering device according to any one of claims 1 to 4, 8 and 9,
characterized in that at least one circumferential portion of the inner wall of said hollow cylinder is expanded in the area of the front end section of said hollow cylinder (24, 28), so that a passage (26, 39) is permitted in said area on the edge of said piston (20, 29).

## Revendications

1. Dispositif de dosage pour l'amenée de deux liquides, desquels au moins un est dosé, caractérisé par un cylindre creux (1, 24, 28), à la paroi interne duquel un piston translatable (2, 20, 29) est adjacent en assurant une étanchéité aux liquides, qui limite une chambre de cylindre avant et une chambre de cylindre arrière (3, 4 ; 31, 30), une ouverture d'entrée (7, 22, 34) pour le premier liquide dans la région arrière du cylindre creux et une ouverture (16, 38) dans la région avant du cylindre creux, à travers laquelle le deuxième liquide dosé est amenable pour remplissage dans la chambre de cylindre avant (3, 31) et hors de laquelle, les deux liquides, par suite de l'avancée du piston, qui est rappelé par la pression du premier liquide agissant sur le côté arrière du piston où la succion agissant sur le côté avant du piston, peuvent sortir, et un dispositif de passage (12, 25, 39) pour le premier liquide, qui, dans une position prédéterminée du piston, permet la sortie du premier liquide dans la chambre de cylindre avant (3, 31) ou bien hors de l'ouverture avant (16, 38) du cylindre creux.

2. Dispositif de dosage selon la revendication 1, caractérisé en ce que le passage dans la chambre de cylindre avant est libéré dans la position avancée du piston (2, 20, 29) .

3. Dispositif de dosage selon la revendication 1 ou 2, caractérisé en ce que, entre le fond de cylindre creux avant (37) et le piston (29), un dispositif de ressort (42) est agencé, qui est comprimé, lorsque le piston est avancé dans la région du dispositif de passage (39), et dont la force de ressort repousse à nouveau le piston dans une région, dans laquelle celui-ci est adjacent de façon étanche à la paroi intérieure de cylindre, lorsque le piston n'est plus sollicité en direction d'avance par pression ou succion de liquide.

4. Dispositif de dosage selon une des revendications 1 à 3, caractérisé en ce qu'avec le piston (2, 20) une tige de traction (6, 21) est reliée, qui, dans la position avancée du piston, fait saillie avec sa section d'extrémité arrière hors d'un perçage de passage ( 7 ) dans la paroi frontale arrière (8) du cylindre creux (1, 24).

5. Dispositif de dosage selon la revendication 4, caractérisé en ce que la tige de traction pénétrant la paroi frontale arrière (8) du cylindre creux (1) est un tube (6) creux, qui est reliable avec un conduit (10), qui reste en liaison avec une source du premier liquide.

6. Dispositif de dosage selon une des revendications 1 à 5, caractérisé en ce que le piston (2) présente un perçage de passage pourvu d'un organe d'obturation (13), qui permet le passage du premier liquide dans la chambre de cylindre avant (3), lorsque l'organe d'arrêt (13) est déplacé dans une position d'ouverture, que l'organe d'arrêt est un corps d'obturation (13), qui est sollicité, par un dispositif de ressort (14), en direction de la paroi frontale avant (15) du cylindre creux (1), dans la position d'obturation, et que dans la position avancée du piston (2) le corps d'obturation (13) vient en contact sur une partie en saillie (19) restant fixe, qui pousse le corps d'obturation dans la position d'ouverture.

7. Dispositif de dosage selon la revendication 6, caractérisé en ce que la partie en saillie est un élément pointu (19) installé dans la région de l'ouverture avant (16) du cylindre creux (1), qui présente au moins un perçage de passage (20), qui reste en liaison avec l'ouverture (16).

8. Dispositif de dosage selon une des revendications 1 à 7, caractérisé en ce qu'une ouverture (22, 34) est constituée pour l'entrée du premier liquide dans la chambre de cylindre arrière (4, 30).

9. Dispositif de dosage selon les revendications 4 et 8, caractérisé en ce que le perçage de passage (7) pénétré par la tige de traction (21), dans la paroi frontale arrière du cylindre creux (24), est étanché avec étanchéité aux liquides vis-à-vis de la tige de traction.

10. Dispositif de dosage selon l'une des revendications 1 à 4, 8 et 9, caractérisé en ce que, dans la région de la section d'extrémité avant du cylindre creux (24, 28), au moins une section périphérique de la paroi intérieure du cylindre creux est élargie, de sorte que dans cette région, un passage (26, 39) est libéré au bord du piston (20, 29).
